# EUROPÄISCHE PATENTSCHRIFT

(11) **EP 0 327 144 B1**
(45) Veröffentlichungstag und Bekanntmachung des Hinweises auf die Patenterteilung: **07.06.1995**
(21) Anmeldenummer: 89106812.4
(22) Anmeldetag: 28.11.1985
(51) Int. Cl.: A61B 10/00, G01N 1/28

(54) **Probenaufnahmegefäss für pastöses Probenmaterial und Verfahren zur Verarbeitung von pastösem Probenmaterial**
Sample holder for pasty sample and method for processing pasty samples
Récipient pour recevoir un échantillon pâteux et procédé de traitement d'échantillons pâteux

(43) Veröffentlichungstag der Anmeldung: 09.08.1989
(62) Teilanmeldung aus: 85115102.7
(73) Patentinhaber: Szabados, Andreas, Dr.med., D-82031 Grünwald (DE)
(72) Erfinder: Szabados, Andreas, Dr.med., D-82031 Grünwald (DE)
(74) Vertreter: Prechtel, Jörg, Dipl.-Phys. Dr.

(56) Entgegenhaltungen:
- EP-A- 0 014 179
- DE-A- 3 347 464
- DE-A- 3 427 114
- GB-A- 1 548 026
- US-A- 3 300 051
- US-A- 3 701 434
- US-A- 3 905 895
- US-A- 4 032 437

## Beschreibung

Die Erfindung betrifft ein Probenaufnahmegefäß mit einem Rühreinsatz zur Vermischung von Flüssigkeiten oder von Flüssigkeiten und Feststoffen.

Ein Probenaufnahmegefäß mit den Merkmalen des Oberbegriffs des Anspruchs 1 ist bekannt (EP-A-0014179). Das Verwirbelungselement wird hierbei von einem Siebboden am unteren Endes eines hohlzylindrischen Einsatzteils gebildet. Ein in den Figuren dargestellter Wulst bzw. nicht dargestellte Dichtlippen am Außenumfang des Einsatzes im Bereich des Siebbodens liegen abdichtend an der Innenumfangsfläche des Probenaufnahmegefäßes an.

Der Erfindung liegt die Aufgabe zugrunde, ein Probenaufnahmegefäß mit einem Rühreinsatz zur Vermischung von Flüssigkeiten oder von Flüssigkeiten und Feststoffen bereitzustellen, welches eine intensive Vermischung bei geringem baulichen Aufwand und einfachster Handhabung ohne die Gefahr von Spritzern oder dergleichen sicherstellt.

Zur Lösung dieser Aufgabe werden die Merkmale des Anspruchs 1 vorgeschlagen.

Es hat sich gezeigt, daß bei dieser Art der Vermischung die Flüssigkeitsoberfläche angenähert in Ruhe bleibt, so daß Spritzer ausgeschlossen sind. Die Wirbel führen schnell und zuverlässig zur gewünschten intensiven Vermischung. Im Falle des eingangs beschriebenen Probenaufnahmegefäßes wird das Verwirbelungselement vom mit den Sieböffnungen versehenen Probenbecher gebildet.

Die Ansprüche 2 bis 4 beschreiben bevorzugte Ausgestaltungen der Erfindung.

Die Erfindung betrifft ferner ein Verfahren zur Verarbeitung von pastösem Probenmaterial, insbesondere infektiösem Material, wie z.B. Stuhl, mit Hilfe des vorstehend beschriebenen Probenaufnahmegefäßes, wobei man die Probe
a) in einer ersten Flüssigkeit, ggf. Fixierlösung, insbesondere MIFC-Lösung, suspendiert,
b) filtriert und
c) das Filtrat ggf. zentrifugiert.

Ein Verfahren dieser Art ist bekannt (Josef Boch, Rudolf Supperer "Veterinärmedizinische Parasitologie", Verlag Paul Parey, Berlin und Hamburg, 1983). Hierbei wird die Stuhlprobe mit Hilfe des löffelförmigen Probenaufnahmebechers aufgenommen und in das sonst leere Probenaufnahmegefäß gesteckt. Anschließend wird das Probenaufnahmegefäß ins Labor geschickt und dort geöffnet und die Stuhlprobe in eine die erste Flüssigkeit enthaltendes Gefäß gegeben und mit einem elektrischen Rührstab intensiv gemischt. Mit Hilfe einer Pipette wird der Suspension ein Probenvolumen entnommen und in einen, eine Gaze tragenden Filtertrichter oberhalb eines Filtratgefäßes gegossen. Das Filtratgefäß wird ggf. zentrifugiert.

Die unangenehme, ggf. infektiöse Probe bzw. Probenlösung wird also mehrfach von Hand aus einem Gefäß in ein anderes gebracht.

Um demgegenüber die Durchführung des Verfahrens für die betroffenen Personen wesentlich einfacher und weniger unangenehm zu gestalten, werden die Maßnahmen des Anspruchs 5 vorgeschlagen.

Da erfindungsgemäß die Probe sogleich nach der Entnahme mit der ersten Flüssigkeit in Kontakt gebracht wird, ergibt sich eine augenblickliche Fixierung des Probenmaterials, so daß eine Zersetzung der interessierenden Teilchen bis zur Laborbehandlung unterbunden wird. Auch wird zugleich eine Geruchsentwicklung unterbunden sowie die Entwicklung von Gasen, welche eine Explosion des Probenaufnahmegefäßes während des Transports zur Folge haben könnte. Die Probenflüssigkeit bleibt im Probenaufnahmegefäß bis zum Aufsetzen des Filterkörpers; zum Filtrieren muß das mit dem Filterkörper versehene Probenaufnahmegefäß lediglich um 180° um eine horizontale Achse gedreht und dann geschüttelt werden. Falls ein Zentrifugieren erwünscht ist, kann hierzu das Filtratgefäß unmittelbar in die Zentrifuge eingesetzt werden. Die anschließende Untersuchung der Flüssigkeiten im Filtratgefäß bzw. der im Filter zurückgehaltenen Teilchen ist für die entsprechende Person nicht mehr mit unangenehmen Eindrükken verbunden.

Das mit den Durchgangsöffnungen versehene und mit der Innenumfangsfläche des Probenaufnahmegefäßes einen Ringspalt mit der angegebenen lichten Weite bildende wandartige Verwirbelungselement sorgt für eine zuverlässige Feinverteilung und intensive Durchmischung, insbesondere im Labor vor der Filtrierung, wozu man den Probenaufnahmebecher mehrfach im Gefäßinnenraum auf und ab bewegt, vorzugsweise durch entsprechende Handhabung eines über einen Stiel mit dem Verwirbelungselement verbundenen Gefäßdeckels. Von dieser einfachen Handhabung abgesehen, ergibt sich der Vorteil, daß keine unter Umständen sogar zu sterilisierenden Hilfsmittel (Wattestäbchen) zum anfänglichen Einrühren und späteren intensiven Durchmischen erforderlich sind. Schließlich ist auch eine Kontamination der Umgebung sowohl bei der anfänglichen Suspendierung der Probe in der ersten Flüssigkeit als auch beim späteren Durchmischen nicht zu befürchten. Es ergibt sich ein geschlossenes Verarbeitungssystem, bei welchem die Belastung der verarbeitenden Personen (Kontamination, Lösungsmitteldämpfe) auf ein Minimum reduziert ist gegenüber den bisher üblichen "offenen" Verarbeitungsmethoden. Auch wird durch die intensive Durchmischung des Materials die Zuverlässigkeit der Methode gesteigert.

Durch die erfindungsgemäße Auf- und Abbewegung des Verwirbelungselements innerhalb der Flüssigkeit erreicht man, daß sich die Mischung aus Probenmaterial und Suspendierflüssigkeit durch die einzelnen Durchgangsöffnungen sowie durch den Ringspalt in Form feiner Strömungsfäden hindurchbewegt, mit dem Ergebnis einer intensiven gleichmäßigen, feinen Durchmischung des Probenmaterials mit dar Suspendierflüssigkeit. Auf Grund der Inkompressibilität der Flüssigkeit und der Feinheit der Strömungsfäden und der Wirbel bleibt bei dem Anheben und Absenken des Gefäßdeckels der Flüssigkeitspegel praktisch vollkommen ruhig. Die Gefahr der Kontamination der Umgebung durch Spritzer ist vernachlässigbar.

Die US-A-4,032,437 zeigt ein Probenaufnahmegefäß mit einem Gefäßdeckel sowie einem Gefäßboden mit zum Gefäßdeckel hin offenem Probenaufnahmebecher. Der Kolben gemäß dem U.S.-Patent ist in Gleitpassung in das Gefäß eingepaßt, wohingegen bei der Erfindung ein Ringspalt zwischen dem Umfangsrand des Verwirbelungselements und der Innenumfangsfläche des Probenaufnahmegefäßes vorgesehen ist, welcher zusätzlich für intensive Durchmischung sorgt. Schließlich ergibt sich auf Grund der bevorzugten Halterung des Verwirbelungselements am Deckel zum einen eine Reduzierung der Anzahl der erforderlichen Bauelemente und zum anderen eine Erhöhung der Zuverlässigkeit in der Handhabung, da beim Schließen des Deckels und anschließendem Wiederöffnen des Deckels selbsttätig die Durchmischung mit der Suspendierflüssigkeit erfolgt.

Aus der DE-A-32 18 079 ist ein Rotor innerhalb eines Bechers bekannt, welcher durch Drehung mit ausreichend hoher Geschwindigkeit Zellanhäufungen im Bereich zwischen Rotor und Becher mit Hilfe entsprechender Scherkräfte aufbricht. Der lichte Abstand zwischen Rotor und Becher beträgt 4 mm. Bei der Erfindung dagegen wird eine Durchmischung durch Axialverschiebung des mit Sieböffnungen versehenen Probenaufnahmebechers innerhalb des Gefäßes erzielt.

Die Erfindung wird im folgenden an Hand der Zeichnung an bevorzugten Ausführungsbeispielen erläutert. Es zeigt:
- Fig. 1: eine seitliche Schnittansicht einer ersten erfindungsgemäßen Ausführungsform eines Probenaufnahmegefäßes vor dem Aufsetzen des Gefäßdeckels;
- Fig. 2: die Anordnung nach Fig. 1 mit aufgesetztem Gefäßdeckel;
- Fig. 3: das Probenaufnahmegefäß gemäß Fig. 1 und 2 vor dem Aufsetzen eines Filterkörpers sowie eines Filtratgefäßes;
- Fig. 4: das mit dem Filterkörper und dem Filtratgefäß versehene, zum Filtrieren verwendete Probenaufnahmegefäß gemäß Fig. 3;
- Fig. 5: eine seitliche Schnittansicht einer zweiten Ausführungsform eines Probenaufnahmegefäßes mit teilweise eingeschraubtem Gefäßdeckel;
- Fig. 6: eine Filtrieranordnung ähnlich Fig. 4;
- Fig. 7: das Detail A in Fig. 6 und
- Fig. 8: einen vergrößerten Ausschnitt eines in der Anordnung gemäß Figuren 6 und 7 eingesetzten Filtergewebes (Blickrichtung B in Fig. 6).

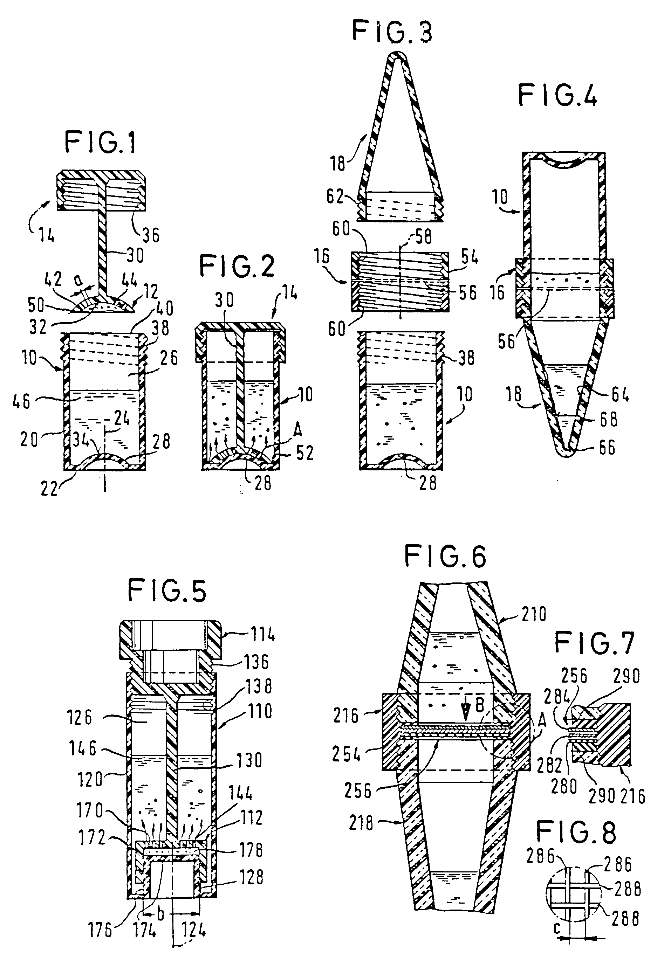

Das in den Figuren 1 bis 4 dargestellte Probenaufnahmegefäß 10 kann wahlweise mit einem in den Figuren 1 und 2 erkennbaren, einen Probeaufnahmebecher 12 aufweisenden Gefäßdeckel 14 oder, ohne daß der Gefäßinhalt umzuschütten ist, mit einem Filterkörper 16 zum Filtrieren des Gefäßinhalts entsprechend Figuren 3 und 4 verwendet werden. Das Probenaufnahmegefäß 10 weist eine hohlzylindrische Gefäßseitenwand 20 sowie einen Gefäßboden 22 auf. Der Gefäßboden 22 ist mit einer zur Gefäßachse 24 zentrisch angeordneten, vom Gefäßinnenraum 26 ausgesehen konvexen, im wesentlichen kalottenförmigen Erhebung 28 ausgeformt. Der über einen Stiel 30 mit dem Gefäßdeckel 14 verbundene Probenaufnahmebecher 12 ist komplementär zur Erhebung 28 gewölbt. Die Länge des Stiels 30 ist nun derart festgelegt, daß bei vollständig aufgeschraubtem Gefäßdeckel 14 gemäß Fig. 2 die Becherinnenseite 32 vollflächig an der Oberseite 34 der Erhebung 28 anliegt. Der angenähert hutförmige Deckel 14 ist mit einem Innengewinde 36 versehen, welches auf ein Außengewinde 38 der Becherseitenwand 20 im Bereich der Becheröffnung 40 aufschraubbar ist.

Die Becherwand 42 des Probenaufnahmebechers 12 ist, über die gesamte Wand verteilt, mit Sieböffnungen 44 versehen mit einer lichten Weite a zwischen 0,5 und 2 mm, am besten etwa 1 mm.

Zur Entnahme einer Probe wird entweder mit Hilfe eines Spatels oder dergl. die Probe in den Probenaufnahmebecher 12 gestrichen oder die Probe unmittelbar mit Hilfe des Probenaufnahmebechers aufgenommen. Aufgrund des vorgegebenen Probenaufnahmebecher-Volumens können auch quantitative Messungen durchgeführt werden. Vorher ist in das Probenaufnahmegefäß 10 eine erste Flüssigkeit 46 eingefüllt worden, die als Fixier- und/oder Transportmedium dient. Im Falle einer Anwendung der MIFC-Technik besteht die erste Flüssigkeit aus Formalin-Wasser-Glycerin zuzüglich Merthiolat (Thimerosal).

Der Gefäßdeckel 14 wird mit dem Probenaufnahmebecher 12 voraus auf das Probenaufnahmegefäß 10 aufgesetzt und mit diesem verschraubt. Während dieses Schraubvorgangs nähert sich die Becherwand 42 zunehmend der Erhebung 28. Zwischen der Becherwand 20 und dem Umfangsrand 50 der Becherwand 42 ist ein schmaler Ringspalt 52 gebildet mit einer den Wert a nicht überschreitenden Spaltweite. Aufgrund dieses Ringspalts kann beim nach unten Bewegen des Probenaufnahmebechers 12 innerhalb des Probenaufnahmegefäßes 10 die erste Flüssigkeit 46 ohne weiteres am Umfangsrand 50 vorbei nach oben ausweichen. Sobald das Probenmaterial 32 jedoch den Becherboden 22 erreicht und der Probenaufnahmebecher 12 weiterhin nach unten bewegt wird (aufgrund der Aufschraubbewegung des Gefäßdeckels 14), wird es komprimiert und aus den Sieböffnungen 44 sowie dem in Fig. 2 angedeuteten Ringspalt 52 gepreßt. In Fig. 2 sind entsprechende kleine Strömungspfeile mit A bezeichnet. Entsprechend der Sieböffnungsweite a und der Anzahl der Sieböffnungen ergeben sich eine Vielzahl feiner Materialproben-Strömungsfäden (Jets) in die erste Flüssigkeit 46.

Dies führt zu einer feinen Verteilung des Probenmaterials in der ersten Flüssigkeit 46. Probenmaterialteilchen, welche größer als die Sieböffnungsweite a sind, werden, falls möglich, zwischen den beiden wie Stempel aufeinanderdrückenden Teilen- Erhebung 28 und Becherwand 42 - zerdrückt, so daß sie schließlich durch die Sieböffnungen 44 entweichen können. Diejenigen Partikel, welche aufgrund ihrer Härte nicht zerdrückbar sind, bleiben zwischen Becherwand 42 und Erhebung 28. Damit der Gefäßdeckel 14 dennoch vollständig aufgeschraubt werden kann und somit das Probenaufnahmegefäß abdichtet, ist sowohl die Becherwand 42 als auch der Stiel 30 elastisch nachgiebig ausgebildet. Dies wird durch Fertigung dieser Teile aus Polyäthylen erreicht. Alternativ oder zusätzlich kann auch die Erhebung 28 elastisch nachgiebig ausgebildet sein.

Allein durch das Zuschrauben des Gefäßdeckels 14 erreicht man also automatisch die Feinverteilung des Probenmaterials in der ersten Flüssigkeit 46. Durch anschließendes leichtes Schütteln kann man die Suspendierung des Probenmaterials in der ersten Flüssigkeit 46 noch verstärken. Falls erforderlich, kann man bei der Probenentnahme oder später im Labor den Suspendierungsgrad in einfacher Weise noch dadurch erhöhen, daß man durch Anheben und Absenken des Gefäßdeckels 14 den Probenaufnahmebecher 12 innerhalb des Probenaufnahmegefäßes auf und ab bewegt. Es ergibt sich eine intensive Durchmischung aufgrund der Wirbelbildung im Bereich der Sieböffnungen 44 sowie des Ringspalts 52. Es werden keine zusätzlichen, ggf. eigens zu sterilisierenden Umrühr-Geräte benötigt. Die Gefahr der Kontamination der Umgebung durch Spritzer oder Lösungsmitteldämpfe ist stark reduziert.

Das Probenaufnahmegefäß 10 kann unmittelbar als Transportgefäß zwischen Probenentnahmeort und Labor verwendet werden. Die erste Flüssigkeit 46 verhindert ein Gären des Probenmaterials, so daß es nicht zu einer Explosion des Probenaufnahmegefäßes kommen kann. Ferner unterbindet die erste Flüssigkeit 46 auch eine Geruchsentwicklung bei entsprechendem Probenmaterial. Schließlich kann die erste Flüssigkeit auch für eine Sterilisierung und Fixierung des Probenmaterials sorgen.

Im Labor wird der Gefäßdeckel 14 abgenommen, ggf. eine zweite Flüssigkeit, insbesondere organisches Lösungsmittel (Äther oder Äthylacetat) oder Farbmittel (z.B.Lugolsche Lösung). zugegeben und nochmals durch Auf- und Abbewegung des Probenaufnahmebechers intensiv vermischt. Nunmehr wird zur anschließenden Filtrierung der in den Figuren 3 und 4 erkennbare Filterkörper 16 aufgeschraubt. Der Filterkörper 16 umfaßt einen hohlzylindrischen Filterträger 54, in dessen Durchgang mit Abstand zu beiden Durchgangsenden ein ein- oder mehrlagiger Filter 56 vorgesehen ist. In Richtung der Hohlzylinderachse 58 beidseits des Filters 56 ist je ein Einschraubgewinde 60 vorgesehen zur Verbindung des Filterkörpers 16 mit dem Probenaufnahmegefäß (Außengewinde 38) bzw. dem Filtratgefäß 18 (Außengewinde 62). Das Filtratgefäß 18 kann also vor oder nach dem Aufschrauben des Filterkörpers 16 auf das Probenaufnahmegefäß 10 mit dem Filterkörper 16 verschraubt werden. Der Filterträger 16 kann in besonders einfacher Weise dadurch hergestellt werden, daß man zwei jeweils mit dem Einschraubgewinde 60 versehene Schraubringe miteinander stirnseitig verklebt unter Zwischenlage des Filters 56. Ein spezieller Filteraufbau wird nachfolgend an Hand der Figuren 6 bis 8 noch näher beschrieben.

Nach dem erfolgten Zusammenschrauben der Teile 10, 16 und 18 wird die Anordnung um 180° um eine horizontale Achse in die Lage gemäß Fig. 4 gedreht. Schüttelt man bei der MIFC-Methode zum Parasitennachweis nun die Anordnung in vertikaler Richtung (ca. 15 Sekunden), so erhält man etwa die Hälfte der Suspension als Filtrat im Filtratgefäß 18. Die Filtrierung der restlichen Suspension erreicht man in einfacher Weise dadurch, daß man das obere Ende der Anordnung (d.h. das Probenaufnahmefäß 10) ergreift und zwei-bis dreimal nach abwärts gerichtet schüttelt (wie bei einem Zurückschlagen eines Quecksilber-Fieberthermometers). Es befindet sich nun die gesamte filtrierte Suspension im Filtratgefäß 18. Nun kann das als Einsendegefäß dienende Probenaufnahmegefäß 10 entfernt werden; die auf dem Filter 56 abgelagerten Partikel können gesondert untersucht werden. Die filtrierte Suspension im Filtratgefäß 18 kann nun entsprechenden Untersuchungen zugeführt werden. Im Falle der MIFC-Methode für Parasitologie läßt man dann, wenn keine Zentrifuge vorhanden ist, die Suspension ca. 12 bis 24 Stunden stehen. Falls eine Zentrifuge vorhanden ist, kann man das Filtratgefäß 18 unmittelbar in die Zentrifuge einsetzen und zentrifugieren. In beiden Fällen erhält man dann eine Flüssigkeitsschichtung im Filtratgefäß 18. Bei der MIFC-Methode interessiert lediglich die unterste Schicht innerhalb des sich nach unten konisch verjüngenden Filtratgefäßes 18. Die darüberliegenden Schichten werden vorsichtig dekantiert. Um hierbei ein versehentliches Entweichen auch der interessierenden untersten Schicht zu verhindern, oder zumindest zu erschweren, ist die Gefäßinnenseite 64 zumindest im Bereich der Spitze 66 aufgerauht und/oder mit einer die Haftung des Probenmaterials bzw. Filtrats an der Gefäßinnenseite 64 verbessernden Beschichtung versehen. Besonders bewährt hat sich eine Silikat-Beschichtung 68.

Die verbleibende unterste Schicht wird nunmehr näher untersucht. Hierzu wird ein Tropfen dieser Schicht auf einen Objektträger gebracht, ein Glasdeckel aufgesetzt und mikroskopisch untersucht.

Bei der in Fig. 5 dargestellten zweiten Ausführungsform eines Probenaufnahmegefäßes sind diejenigen Bauelemente, welche ihrer Funktion nach solchen der Ausführungsform gemäß Figuren 1 bis 4 entsprechen, mit denselben Bezugsziffern, jeweils vermehrt um die Zahl 100, versehen. Das demzufolge mit 110 bezeichnete Probenaufnahmegefäß ist im Unterschied zur ersten Ausführungsform mit einem Innengewinde 138 versehen, in welches ein dementsprechend mit einem Außengewinde 136 versehener Gefäßdeckel 114 einschraubbar ist. Der Hauptunterschied zur ersten Ausführungsform liegt jedoch in der unterschiedlichen Gestaltung des Probenaufnahmebechers 112 und dementsprechend der Erhebung 128. Der Probenaufnahmebecher ist mit einem in Bezug auf die Gefäßachse 124 radialen, kreisrunden Becherboden 170 versehen, in welchem die Sieböffnungen 144 ausgeformt sind. Vom Umfangsrand des Becherbodens 170 geht eine hohlzylindrische Becherseitenwand 172 aus. Die hierzu komplementär ausgeformte Erhebung 128 weist demnach einen Erhebungsboden 174 sowie eine hohlzylindrische Erhebungsseitenwand 176 auf, deren Außendurchmesser b etwa dem Innendurchmesser der Becherseitenwand 172 entspricht. Zwischen dem Außenumfang der Becherseitenwand 172 und dem Innenumfang der Gefäßseitenwand 120 befindet sich ein ausreichend großer Spalt, z.B. von etwa 1,5 mm, für das Überströmen der Flüssigkeit 146 beim Nachuntenschieben des Probenaufnahmebechers 112. Der vom Becherboden und von der Becherseitenwand 172 umschlossene Probenaufnahmeraum 178 ist folglich zylindrisch. Beim Einschieben des Probenaufnahmebechers 112 in das Probenaufnahmegefäß 110 gelangt schließlich die Becherseitenwand 172 in Kontakt mit der Erhebungsseitenwand 176. Die Erhebung 128 fährt in der Folge nach Art eines Kolbens in den Probenaufnahmebecher 112 ein und verdrängt das Probenmaterial innerhalb des Probenaufnahmeraums 178. Dieses wird jet-artig durch die Sieböffnungen 144 in die erste Flüssigkeit 146 gepreßt. Bei vollständig aufgeschraubtem Deckel 114 liegt der Becherboden 170 am Erhebungsboden 174 vollflächig an, es sei denn, daß Partikel, wie z.B. Steinchen, den Becherboden vom Erhebungsboden in entsprechendem Abstand halten. Da der Stiel 130 elastisch nachgiebig ist, kann dennoch der Gefäßdeckel 114 vollständig abdichtend aufgeschraubt werden.

Zur Verbesserung der Verteilung des Probenmaterials in der jeweiligen Flüssigkeit innerhalb des Probenaufnahmegefäßes 110 kann man, wie schon an Hand der Figuren 1 und 2 beschrieben, den Probeaufnahmebecher 112 innerhalb des Gefäßinnenraums 126 mehrfach auf und ab schieben.

In einer nicht dargestellten Ausführungsform der Erfindung ist der den Probenaufnahmebecher tragende Stiel lösbar mit dem Gefäßdeckel verbunden, wozu der Gefäßdeckel an seiner Innenseite dementsprechend mit einem Einsteck-Sackloch versehen sein kann.

Der genauere Siebaufbau geht aus den Figuren 6 bis 8 hervor. Bauelemente, welche ihrer Funktion nach solchen in den Figuren 1 bis 3 entsprechen, sind mit denselben Bezugsziffern, jeweils vermehrt um die Zahl 200, versehen. In den Filterträger 216 ist von einer Seite aus das Probenaufnahmegefäß 210 eingeschraubt und von der anderen Seite aus das Filtratgefäß 218. Im nunmehr zu beschreibenden Ausführungsbeispiel haben Filtratgefäß 218 und Probenaufnahmegefäß 210 die gleiche spitz zulaufende Form, da die erfindungsgemäße Filtrierung innerhalb eines geschlossenen Systems auch unabhängig von der Probensuspendierung mit Hilfe entsprechend ausgebildetem Probenaufnahmegefäß und Gefäßdeckel (Erhebung bzw. Probenaufnahmebecher) durchgeführt werden kann, obschon sie, insbesondere bei der MIFC-Technik, mit besonderem Vorteil mit dementsprechend ausgebildetem Probenaufnahmegefäß durchführbar ist.

Der Filter 256 ist insgesamt drei-lagig. Auf ein Stützsieb 280 folgt ein Hauptfilter 282 und anschließend ein Vorfilter 284. Wenigstens eine der Lagen, am besten sämtliche Lagen, werden jeweils von einem Gewebesieb gebildet, also aus einem Sieb mit gewebeartig sich gegenseitig kreuzenden Gewebefäden oder -strängen. In Fig. 8 sind parallel zueinander liegende erste Fäden 286 gezeigt, welche sich rechtwinkelig mit zweiten Fäden 288 kreuzen. Die lichte Maschenweite c zwischen aufeinanderfolgenden Fäden ist entsprechend der jeweiligen Lage (Stützsieb bzw. Hauptfilter bzw. Vorfilter) und der gewünschten, gerade noch durchzulassenden Teilchengröße festgelegt. So wird man beispielsweise zum Abfiltrieren von Bakterien, Viren, Enzymen und Substraten von größeren Parasiten folgende Maschenweiten wählen: Stützsieb 20 - 100 »m; Hauptfilter 3 - 5 »m; Vorfilter 20 - 100 »m. Zum Trennen von Bakterien und Pilzsporen von größeren Partikeln wählt man für die lichte Maschenweite c folgende Werte: Stützsieb 20 - 100 »m; Hauptfilter 0,15 - 0,45 »m; Vorfilter 5 - 20 »m.

Die aus Metall und/oder Kunststoffäden bzw. -strängen gebildeten Gewebefäden verhindern ein Anhaften von Partikeln am Filter, welche an und für sich den Filter passieren sollten. Auch ergibt sich aufgrund der Gewebestruktur eine hohe Trennschärfe (geringe Streuung der lichten Maschenweite bei einem Gewebesieb).

Um die drei Lagen 280 bis 284 zusammenzuhalten und zudem ein Austreten von Flüssigkeit aus der zusammengeschraubten Anordnung der Teile 210, 216, 218 zu verhindern, ist beidseits des drei-lagigen Filters 265 jeweils ein O-Ring 290 eingesetzt, welcher gemäß Fig. 7 rechteckigen Querschnitt aufweist. Mit seinem Außenumfang liegt jeder Ring 290 am Innenumfang des hohlzylindrischen Filterträgers 254 an. An die vom drei-lagigen Filter 256 jeweils abgewandte Stirnseite drückt das Filtratgefäß 218 bzw. das Probenaufnahmegefäß 210. Der mehrteilige Filter 256 kann austauschbar ausgebildet sein, um einen Filterträger 216 durch entsprechende Filterwahl an die verschiedenen Anwendungsbereiche anpassen zu können.

Die Handhabung der Anordnung gemäß Fig. 6 entspricht der der Anordnung gemäß Figuren 3 und 4. Auf das die zu filtrierende Suspension enthaltende Probenaufnahmegefäß 210 ist also der Filterträger 216 samt Filtratgefäß 218 aufzuschrauben und anschließend um 180° um eine horizontale Achse zu drehen. Das anschließende Filtrieren kann durch Schütteln oder Schlagen oder Zentrifugieren der Anordnung gemäß Fig. 6 unterstützt werden. Aufgrund des vollständig abgeschlossenen Systems ist die Kontaminationsgefahr (Spritzer, Lösungsmitteldämpfe oder dergl.) beseitigt. Auch entfällt jedwede Geruchsbelästigung. Die Verarbeitung von unangenehme Gefühle auslösenden Materialien, wie z.B. Stuhlproben, gestaltet sich auf die angegebene Weise wesentlich angenehmer.

Das erfindungsgemäße Probenaufnahmegefäß eignet sich zur schnellen Reinigung oder Steril-Filtration von sämtlichen flüssigen Suspensionen oder Lösungsmitteln, wobei keine nennenswerten Verluste an Probenmaterial auftreten. Für die Filtrieranordnung kann unmittelbar das Transportgefäß bzw. Verarbeitungsgefäß bzw. Lagerungsgefäß verwendet werden, da lediglich der Filterträger samt Filtratgefäß in üblicher Weise, z.B. durch Aufschrauben, aufzusetzen ist. Die Filtriermethode eignet sich zur Anwendung bei empfindlichen Chromatografiesystemen, wie z.B. HPLC, im Bereich der Immunologie, Molekularbiologie, Zellbiologie, Bakteriologie, Virologie oder dergl.

## Patentansprüche

1. Probenaufnahmegefäß mit einem Rühreinsatz zur Vermischung von Flüssigkeiten oder von Flüssigkeiten und Feststoffen, der in dem Probenaufnahmegefäß (10; 110) auf und ab beweglich ist und mit einem, den Gefäßquerschnitt im wesentlichen ausfüllenden, mit Durchgangsöffnungen (14; 114) versehenen, wandartigen Verwirbelungselement (12; 112) versehen ist, **dadurch gekennzeichnet,** daß zwischen dem Außenumfang des Verwirbelungselements (12; 112) und der Innenumfangsfläche des Probenaufnahmegefäßes (10; 110) ein Ringspalt (52) gebildet ist mit einer die lichte Weite (a) der Durchgangsöffnungen im wesentlichen nicht überschreitenden Spaltweite.

2. Probenaufnahmegefäß nach Anspruch 1, **dadurch gekennzeichnet,** daß das Verwirbelungselement (12; 112) über einen Stiel (30; 130) an einem Gefäßdeckel (14; 114) angebracht ist.

3. Probenaufnahmegefäß nach Anspruch 1 oder 2, **dadurch gekennzeichnet,** daß das Verwirbelungselement (12) von einer mit den Durchgangsöffnungen (44) versehenen, zum Gefäßboden (22) hin konkaven Becherwand (42) gebildet ist.

4. Probenaufnahmegefäß nach Anspruch 1 oder 2, **dadurch gekennzeichnet,** daß das Verwirbelungselement (112) von einem mit den Durchgangsöffnungen (44) versehenen, radialen Becherboden (170) gebildet ist, der mit einer hohlzylindrischen Becherseitenwand (172) versehen ist.

5. Verfahren zur Verarbeitung von pastösem Material, insbesondere infektiösem Material, wie z.B. Stuhl, wobei man die Probe in einer ersten Flüssigkeit, vorzugsweise Fixierlösung, am besten MIFC-Lösung suspendiert, filtriert und das Filtrat ggf. zentrifugiert, **dadurch gekennzeichnet,** daß man am Probenaufnahmeort vor der Zugabe der Probe die erste Flüssigkeit in ein Probenaufnahmegefäß einfüllt, daß man bei der Probenentnahme oder im Labor die Probe in der Flüssigkeit dadurch feinverteilt, daß man ein am Gefäßdeckel angebrachtes Verwirbelungselement (12; 112) mehrfach im Gefäß auf und ab bewegt, wobei zwischen dem Außenumfang des wandartigen Verwirbelungselements (12; 112) und der Innenumfangsfläche des Probenaufnahmegefäßes (10; 110) ein Ringspalt (52) gebildet ist mit einer die lichte Weite (a) von Durchgangsöffnungen (14; 114) des Verwirbelungselements (12; 112) im wesentlichen nicht überschreitenden Spaltweite, daß man den Gefäßdeckel abnimmt und an dessen stelle einen Filterkörper samt Filtratgefäß am Probenaufnahmegefäß anbringt und daß man das Probenaufnahmegefäß um 180° um eine horizontale Achse dreht und die Probe filtriert, vorzugsweise unterstützt durch manuelles Schütteln, und daß man ggf. das Filtratgefäß in eine Zentrifuge einsetzt.

6. Verfahren nach Anspruch 5, **dadurch gekennzeichnet,** daß man vor dem Anbringen des Filterkörpers eine zweite Flüssigkeit, insbesondere Lösungsmittel, Flotationsmittel oder Färbemittel, in das Probenaufnahmegefäß einfüllt.

7. Verfahren zum Filtrieren von Suspensionen, insbesondere Bakterien, Viren, Antigene, Enzyme oder Substrate enthaltenden Suspensionen, **dadurch gekennzeichnet,** daß man die Suspension in ein Probenaufnahmegefäß einfüllt, die Suspension dadurch feinverteilt, daß man ein am Gefäßdeckel angebrachtes Verwirbelungselement (12; 112) mehrfach im Gefäß auf und ab bewegt, wobei zwischen dem Außenumfang des wandartigen Verwirbelungselements (12; 112) und der Innenumfangsfläche des Probenaufnahmegefäßes (10; 110) ein Ringspalt (52) gebildet ist mit einer die lichte Weite (a) von Durchgangsöffnungen (14; 114) des Verwirbelungselements (12; 112) im wesentlichen nicht überschreitenden Spaltweite, einen Filterkörper samt Filtratgehäuse aufsetzt und nach einer Drehung dieser Anordnung um 180° um eine horizontale Achse filtriert, ggf. unterstützt durch Schütteln der Anordnung per Hand oder durch Einsetzen in eine Zentrifuge.

## Claims

1. Sample taking receptacle having a stir insert for mixing liquids of liquids and solids which can be moved up and dawn in the sample taking receptacle (10; 110) and is provided with a wall-like vortexing element (12; 112) mostly covering the cross-section of the receptacle and through openings (44;144),
characterised in that
an annular slit (52) is formed between the outer periphery of the vortexing element (12; 112) and the inner peripheral surface of the sample taking receptacle (10; 110) with a slit width not exceeding the width (a) of the through openings.

2. Sample taking receptacle according to Claim 1,
characterised in that the vortexing element (12; 112) is attached to a receptacle lid (14; 114) by means of a stem (30; 130).

3. Sample taking receptacle according to Claim 1 or 2,
characterised in that the vortexing element (12) is formed by a beaker wall (42) provided with through openings (44) and concave towards the receptacle base (22).

4. Sample taking receptacle according to Claim 1 of 2,
characterised in that the vortexing element (112) is formed by a radial beaker base (170) provided with through openings (44) which has a hollow cylindrical beaker side wall (172).

5. Method for processing paste-like material, particularly infectious material such as e.g. stool, whereby the sample is suspended in a first liquid, preferably a fixing solution, best of all a MIFC (Merthiolate Iodine Formol Concentration) solution, filtered and if necessary the filtrate is centrifuged,
characterised in that at the sample taking site a sample taking receptacle is filled with the first liquid before the sample is added, the liquid is finely dispersed when the sample is collected or in the laboratory in that a vortexing element (12; 112) attached to the receptacle lid is moved up and dawn in the receptacle repeatedly, whereby an annular slit (52) is formed between the outer periphery of the wall-like vortexing element (12; 112) and the inner peripheral surface of the sample taking receptacle (10; 110) with a slit width not exceeding the width (a) of the through openings, the receptacle lid is removed and a filter body together with the filtrate receptacle is attached in its place to the sample taking receptacle and that the sample taking receptacle is rotated by 180° about a horizontal axis and the sample is filtered, preferably supplemented by manual shaking and, if necessary, the filtrate receptacle is inserted into a centrifuge.

6. Method according to Claim 5,
characterised in that before the filter body is attached the sample taking receptacle is filled with a second liquid, in particular a solvent, floatation means or colorant.

7. Method for filtering suspensions, in particular bacteria, viruses, antigens, enzymes or suspensions containing substrates,
characterised in that the sample taking receptacle is filled with suspension, the suspension is finely dispersed in that a vortexing element (12; 112) attached to the receptacle lid is moved up and dawn in the receptacle repeatedly, whereby an annular slit (52) is formed between the outer periphery of the wall-like vortexing element (12; 112) and the inner peripheral surface of the sample taking receptacle (10; 110) with slit width not exceeding the width (a) of the through openings (44; 144), a filter body is inserted together with filter housing and filtration occurs after a rotation of the assembly by 180° about a horizontal axis, if necessary supported by shaking the assembly by hand or inserting it into a centrifuge.

## Revendications

1. Récipient destiné à recevoir un échantillon, comportant un dispositif agitateur destiné à mélanger des liquides ou des liquides et des matières solides, qui est susceptible de monter et de descendre dans le récipient (10 ; 110) destiné à recevoir un échantillon, et comportant un élément créant des tourbillons (12 ; 112), du tue paroi, pourvu de passages (14 ; 114), remplissant sensiblement la section du récipient, caractérisé en ce qu'est formée, entre le pourtour extérieur de l'élément créant des tourbillons (12 ; 112) et la surface circonférentielle intérieure du récipient (10 ; 110) destiné à recevoir des échantillons, une fente annulaire (52) dont la largeur ne dépasse pas sensiblement la largeur intérieure (a) des passages.

2. Récipient destiné à recevoir un échantillon selon la revendication 1, caractérisé en ce que l'élément créant des tourbillons (12 ; 112) est monté, par l'intermédiaire d'une tige (30 ; 130) sur un couvercle (14 ; 114) du récipient.

3. Récipient destiné à recevoir un échantillon selon la revendication 1 ou 2, caractérisé en ce que l'élément créant des tourbillons (12) est constitué d'une paroi de godet (42), concave en direction du fond (22) du récipient, pourvue des passages (44).

4. Récipient destiné à recevoir un échantillon selon la revendication 1 ou 2, caractérisé en ce que l'élement créant des tourbillons (112) est constitué d'un fond de godet (170) radial, pourvu des ouvertures (44), qui est pourvu d'une paroi latérale de godet (172) cylindrique creuse.

5. Procédé de traitement de matériau pâteux, en particulier de matériau infectieux, comme par exemple des selles, l'échantillon étant mis en suspension et filtré dans un premier liquide, de préférence une solution de fixation, de préférence une solution MIFC, et le filtrat étant éventuellement centrifugé, caractérisé en ce qu'à l'endroit où est prélevé l'échantillon, avant d'ajouter l'échantillon, le premier liquide est versé dans un récipient destiné à recevoir un échantillon, en ce que, lorsque l'échantillon est prélevé ou au laboratoire, on disperse l'échantillon dans le liquide en faisant monter et descendre plusieurs fois dans le récipient un élément créant des tourbillons (12 ; 112) monte sur le couvercle du récipient, une fente annulaire (52) étant formée entre le pourtour extérieur de l'élément créant des tourbillons (12 ; 112), du type paroi, et la surface circonférentielle intérieure du récipient (10 ; 110) destiné à recevoir un échantillon, la largeur de la fente ne dépassant pas sensiblement la largeur intérieure (a) de passages (14 ; 114) de l'élément créant des tourbillons (12 ; 112), en ce que l'on retire le couvercle du récipient et que l'on dispose, à sa place, sur le récipient destiné à recevoir un échantillon, un corps de filtre ainsi qu'un récipient à filtrat, et en ce que l'on fait tourner le récipient destiné à recevoir un échantillon de 180° autour d'un axe horizontal et que l'on filtre l'échantillon, opération qui est, de préférence, soutenue par une agitation manuelle, et en ce que l'on place, le cas échéant, le récipient à filtrat dans une centrifugeuse.

6. Procédé selon la revendication 5, caractérisé en ce que l'on verse, avant de placer le corps de filtre, un deuxième liquide, en particulier un solvant, un réactif de flottation ou un colorant, dans le récipient destiné à recevoir un échantillon.

7. Procédé de filtration de suspensions, en particulier de suspensions contenant des bactéries, des virus, des antigènes, des enzymes ou des substrats, caractérisé en ce que l'on verse la suspension dans un récipient destiné à recevoir un échantillon, en se que l'on disperse la suspension en faisant monter et descendre plusieurs fois dans le récipient un élément créant des tourbillons (12 ; 112) monté sur le couvercle du récipient, une fente annulaire (52) étant formée entre le pourtour extérieur de l'élément créant des tourbillons (12 ; 112), du type paroi, et la surface circonférentielle intérieure du récipient (10 ; 110) destiné à recevoir un échantillon, la largeur de la fente ne dépassant pas sensiblement la largeur intérieure (a) de passages (14 ; 114) de l'élément créant des tourbillons (12 ; 112), en ce que l'on appose un corps de filtre ainsi qu'un boîtier de filtre et, après avoir fait effectuer à ce dispositif une rotation de 180° autour d'un axe horizontal, en qu'on filtre, opération qui est, le cas échéant, soutenue par une agitation manuelle du dispositif,ou par un placement dans une centrifugeuse.
